(19) Europäisches Patentamt
European Patent Office
Office européen des brevets

(11) **EP 1 086 089 B1**

(12) **EUROPÄISCHE PATENTSCHRIFT**

(45) Veröffentlichungstag und Bekanntmachung des Hinweises auf die Patenterteilung:
**16.01.2002 Patentblatt 2002/03**

(21) Anmeldenummer: **99955265.6**

(22) Anmeldetag: **21.05.1999**

(51) Int Cl.⁷: **C07D 307/32**

(86) Internationale Anmeldenummer:
**PCT/EP99/03506**

(87) Internationale Veröffentlichungsnummer:
**WO 99/62895 (09.12.1999 Gazette 1999/49)**

# (54) VERFAHREN ZUR HERSTELLUNG VON BUTYROLACTONEN

METHOD FOR PRODUCING BUTYROLACTONES

PROCEDE DE PREPARATION DE BUTYROLACTONES

(84) Benannte Vertragsstaaten:
**BE DE ES FR GB IT NL**

(30) Priorität: **03.06.1998 DE 19824534**

(43) Veröffentlichungstag der Anmeldung:
**28.03.2001 Patentblatt 2001/13**

(73) Patentinhaber: **BASF AKTIENGESELLSCHAFT**
**67056 Ludwigshafen (DE)**

(72) Erfinder:
- **BRUNNER, Melanie**
  **D-67105 Schifferstadt (DE)**
- **RAHN, Ralf-Thomas**
  **D-68167 Mannheim (DE)**
- **RHEUDE, Udo**
  **D-67166 Otterstadt (DE)**
- **HENKELMANN, Jochem**
  **D-68165 Mannheim (DE)**

(56) Entgegenhaltungen:
**EP-A- 0 429 963** **EP-A- 0 584 720**
**WO-A-97/07111** **DE-A- 3 201 723**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 086 089 B1

**Beschreibung**

Die Erfindung betrifft ein Verfahren zur Herstellung von Butyrolactonen durch Carbonylierung von Alkinen mit Kohlenmonoxid in Gegenwart von Wasser an einen Rhodiumkatalysator und Abscheidung des Rhodiumkatalysators durch unmittelbare Hydrierung des Carbonylierungsreaktionsgemisches, Abtrennung und Rückführung des Katalysators und Gewinnung des Butyrolactons aus dem Filtrat.

Aus WO 97/07111 ist bekannt, Alkine mit Kohlenmonoxid und Wasser in Gegenwart von Übergangsmetallkatalysatoren insbesondere Rhodium zu Butyrolactonen umzusetzen. Dabei bilden sich als Zwischenprodukte 2(5H)-Furanone wie dies durch Takaski Joh et. al. in Inorganica Chi. Acta, 220 (1994) 45 beschrieben ist. Die 2(5H)-Furanone können durch Hydrierung in situ oder getrennt in die Butyrolactone überführt werden. Die vollständige Hydrierung zu den Butyrolactonen erfolgt dabei entweder durch Verschärfen der Carbonylierungsreaktion in Gegenwart von Wasser, bei der sich Wasserstoff durch das Wassergasgleichgewicht bilden kann, durch Zusatz von Wasserstoff bei der Carbonylierung oder durch nachträgliche Hydrierung der isolierten 2(5H)-Furanone.

Es hat sich nun gezeigt, daß bei der Herstellung der Butyrolactone gemäß WO 97/07111 die Katalysatorabtrennung und Wiederverwendung unbefriedigend ist. Man kann zwar nach Aufarbeitung des Syntheseaustrags, d.h. Gewinnung der Butyrolactone durch Destillation, den im Destillationsrückstand gelöst verbleibenden Katalysator wiederverwenden. Doch hat dies den Nachteil, daß zum einen der Katalysator nicht vom Destillationsrückstand abgetrennt werden kann und somit eine Anreicherung vom Hochsiedern bei mehrfacher Rückführung erfolgt und zum anderen der mehrfach wiederverwendete Katalysator eine deutlich verringerte Selektivität und Aktivität bezüglich der gewünschten Umsetzung zu Butyrolactonen aufweist.

Es bestand daher die Aufgabe, ein Verfahren vorzuschlagen, um den Rhodium-Carbonylierungskatalysator möglichst vollständig in einfacher Weise und unter Erhaltung der Aktivität zu isolieren und ihn in die Reaktion zurückführen zu können.

Auf dem Gebiet der Hydroformylierung sind zahlreiche Verfahren zur Abtrennung von Rhodium beschrieben. Gemäß US 4,400,547 setzt man nach Beendigung der Reaktion dem Oxorohprodukt eine komplexbildende Verbindung wie Triphenylphosphin zu und destilliert den Aldehyd als Produkt ab. Anschließend wird der Destillationsrückstand mit Sauerstoff behandelt, um den Ligand aus der Komplexverbindung wieder abzuspalten und Rhodium in aktiver Form zurückzugewinnen. Eine Trennung von Rhodium und Destillationsrückstand ist auf diese Weise jedoch nicht möglich.

Die Abtrennung von Rhodium aus hochsiedenden Hydroformylierungsrückständen wird auch in der US-PS 35 47 964 beschrieben. Hierzu behandelt man die Rückstände in Gegenwart von Säuren wie Ameisensäure, Salpetersäure oder Schwefelsäure mit Wasserstoffperoxid, um das Rhodium in eine wasserlösliche Form zu überführen. Der technischen Anwendung sind wegen des Wasserstoffperoxidpreises und seiner problematischen Handhabung jedoch Grenzen gesetzt.

Aus EP-A 584 720 B1 ist ferner ein gegebenenfalls zweistufiges Extraktionsverfahren bekannt bei dem Rhodium als Komplexverbindung aus den Destillationsrückständen von Produkten der Oxosynthese zurückgewonnen wird. In der ersten Stufe werden die Rückstände mit Sauerstoff oder einem Sauerstoff enthaltenden Gas in Gegenwart einer Monocarbonsäure und des Alkalisalzes der Monocarbonsäure behandelt. Daraufhin extrahiert man den Rückstand mit Wasser, wobei das als wasserlösliche Verbindung vorliegende Rhodium in die wäßrige Phase übergeht. Dieses Verfahren ist im vorliegenden Fall nicht anwendbar, weil sich gezeigt hat, daß eine quantitative Extraktion des Rhodiumhaltigen Komplexes aus dem Reaktionsaustrag der Butyrolacton-Synthese nur schlecht möglich ist.

Andere Verfahren z.B. gemäß DE-A 43 26 076 betreffen die Wiedergewinnung von Rhodium durch Veraschen, d.h. Verbrennen der organischen Bestandteile. Das so gewonnene Rhodium kann nicht unmittelbar wiederverwendet werden, sonder bedarf einer weiteren Aufarbeitung.

Somit bestand nicht die Möglichkeit das vorliegende Problem mit Methoden des Standes der Technik in wirtschaftlich befriedigender Weise zu lösen.

Es wurde nun überraschenderweise gefunden, daß man in sehr einfacher Weise den Katalysator in aktiver und unmittelbar wiederverwendbarer Form zurückgewinnen kann, mit einem Verfahren zur Herstellung von Butyrolactonen der allgemeinen Formel I

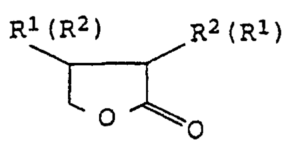

I,

in der die Substituenten $R^1$ und $R^2$ für Wasserstoff, Alkyl-, Hydroxyalkyl- oder gegebenenfalls mit Halogen, Alkoxy und/oder Alkyl inerte Substituenten tragende Aryl- und Trialkylsilylgruppen stehen, durch Umsetzung von Alkinen der all-

gemeinen Formel II

$$R^1—C\equiv C—R^2 \qquad II,$$

in der $R^1$ und $R^2$ die oben angegebene Bedeutung haben, mit vorzugsweise mindestens 4 Äquivalenten Kohlenmonoxid und vorzugsweise mindestens 2 Äquivalenten Wasser in Gegenwart eines homogenen Rhodium-katalysators unter Drücken von 20 bis 300 bar und Hydrierung der intermediär gebildeten 2(5H)-Furanone, wenn man

a) das Reaktionsgemisch der Carbonylierung unmittelbar, (d.h. ohne Abtrennung des Zielprodukts 2(5H)-Furanon gegebenenfalls im Gemisch mit Butyrolacton) mit Wasserstoff bei Temperaturen von 150 bis 250°C vorzugsweise 180 bis 230°C und Drücken von 100 bis 300 vorzugsweise 150 bis 250 bar umsetzt,

b) den ausgefallenen Katalysator abtrennt, und in die Carbonylierungsreaktion zurückführt und

c) aus dem vom Katalysator befreiten Reaktionsgemisch das Butyrolacton durch Destillation gewinnt.

Es war dabei überraschend, daß es so gelingt, den Katalysator in über 99 % der eingesetzten Menge zurückzugewinnen und daß auch nach häufiger Wiederholung der Operation oder nach längerdauernder kontinuierlicher Durchführung die Selektivität und Aktivität des Katalysators erhalten bleibt.

In WO 97/07111, Seite 7, 5. Absatz ist zwar angegeben, daß es sinnvoll sein kann, nur Gemische mit überwiegend Butyrolacton herzustellen. Diese Gemische können dann "direkt" der Hydrierung zugeführt werden oder in ihre Einzelkomponenten Butyrolacton und Furanon, aufgetrennt werden. Es wird jedoch keine Lehre gegeben, daß bei Hydrierung der noch den Katalysator enthaltenden Reaktionsgemisches unter bestimmten Bedingungen der Katalysator ausfällt und dann abgetrennt und in die Reaktion zurückgeführt werden kann. Vielmehr wird im einzigen einschlägigen Beispiel (8) erst das vom Katalysator befreite 2(5H)Furanon bei Normaldruck und Raumtemperatur über einem speziellen Hydrierkatalysator, hier einem Palladiumkatalysator, hydriert.

Für die Stufe (a) des erfindungsgemäßen verfahrens wird das Carbonylierungsreaktionsgemisch unmittelbar, d.h. ohne Abtrennung der intermediar gebildeten 2(5H) Furanone und der bereits gebildeten Butyrolactone, nach Entspannung des restlichen CO-Druckes einer Hydrierung mit Wasserstoff bei Drücken von 20 bis 300 bar, vorzugsweise 150 bis 250 bar und insbesondere 180 bis 220 bar unterworfen. Dabei wird der Rhodiumcarbonyl-Katalysator in eine unlösliche Form, vermutlich durch Entfernung eines Teils der Carbonylgruppen und vermutlich durch Bildung eines Rhodium-Clusters, überführt und die 2(5H)Furanone zu dem entsprechenden Butyrolactonen hydriert. Die Reaktionstemperatur für die Hydrierung beträgt im allgemeinen 0 bis 300°C, bevorzugt 50 bis 150°c, die Reaktionszeit für die Hydrierung im allgemeinen 0,1 bis 24 h, bevorzugt 0,5 bis 5 Stunden.

Bei der Umsetzung fällt ein Katalysator-haltiger Feststoff an, der mit den gängigen Methoden wie z.B. Filtrieren, Zentrifugieren oder Sedimentieren abgetrennt werden kann. Gleichzeitig wird ggf. vorhandenes 2(5H)-Furanon vollständig und mit sehr hoher Selektivität zum Butyrolacton hydriert. Der ausgefällte Katalysator kann ohne signifikante Aktivitäts- und Selektivitätsabnahme für erneute Butyrolacton-Synthesen eingesetzt werden. Die Sequenz Carbonylierung-Katalysatorausfällung-Katalysatorabtrennung-Carbonylierung kann dabei mehrfach wiederholt werden. Die Ausfällung des Katalysators ist jeweils nahezu quantitativ; homogen gelöst im Reaktionsaustrag verbleibt nur weniger als 1 % des anfänglich eingesetzten Rhodiums.

Das erfindungsgemäße Verfahren kommt für alle für die beschriebene Butyrolactonsynthese geeigneten homogenen Rhodium-katalysatore in Betracht. Bevorzugt sind die Katalysatoren $Rh_6(CO)_{16}$, $Rh_4(CO)_{12}$, $Rh(CO)_2acac$, $[codRhCl]_2$, $RhCl_3*3H_2O$ und $Rh(OAc)_3$ zu nennen.

Die Menge an eingesetztem Katalysator liegt in der Regel bei 0,01 bis 10 mmol pro Mol Alkin. Die abgeschiedenen, abgetrennten und rückgeführten Katalysatoren liegen als Verbindungen unbekannter Struktur vor; sie gehen jedoch in der Carbonylierungsstufe wieder in Lösung und erlangen wieder ihre frühere Aktivität. In dem Maße, wie die Abtrennung unvollständig ist, wird die Reaktionslösung durch frischen Katalysator ergänzt.

In der bevorzugten kontinuierlichen Fahrweise der Carbonylierungsverfahrens wie sie im einzelnen in WO 97/07111 beschrieben ist, kann bei der Umsetzung mit Acetylen dieses in einem Sättiger in einem Lösungsmittel gelöst werden. Der Katalysator, Wasser sowie Zusatzstoffe werden ebenfalls gelöst. Diese Lösung wird auf den Reaktionsdruck verdichtet und in den Reaktor gepumpt. CO und weiteres Acetylen werden über eine Gasdüse in den Reaktor gebracht. Der Reaktonsaustrag wird entspannt und die Gasphase kann - gegebenenfalls nach einem Reinigungsschritt - zurück in den Reaktor geführt werden. Der Reaktionsaustrag wird erfindungsgemäß auf den Reaktionsdruck der Hydrierung verdichtet und in einen druckfesten Reaktor, wie einen Rühr-, Rohr- oder Schlaufenreaktor gepumpt. Wasserstoff wird über eine Gasdüse in den Reaktor eingebracht und der Reaktionsaustrag wird nach Verlassen des Reaktors entspannt. Mittels einer der gängigen Methoden wie Filtration, Querstromfiltration, Sedimentation oder Zentrifugation, kann der bei der Hydrierung ausgefällte Rhodiumhaltige Niederschlag kontinuierlich oder diskontinuierlich abgetrennt werden. Der Feststoff wird dann erneut als Katalysator eingesetzt. Die Wasserstoff-Gasphase kann - gegebenenfalls nach einem Reinigungsschritt - zurück in den Hydrierreaktor geführt werden.

Die Carbonylierung der Alkine in Gegenwart von Wasser erfolgt in an sich bekannter Weise, wie dies in WO 97/07111 eingehend beschrieben ist.

Dabei können die Alkine der Formel II gleiche oder verschiedene Substituenten tragen. Sind die Substituenten verschieden, können sie im Reaktionsprodukt jeweils in 3- bzw. 4-Stellung eingebaut werden. In diesen Fällen sind also Isomerengemische zu erwarten. Aus diesem Grund sind in Formel I die Substituenten alternativ in 3-Position wie auch für Verbindungen, die in 4-Position den Substituenten $R^1$ bzw. $R^2$ tragen.

Alkylsubstituierte Alkine II tragen als Alkylgruppen z.B. $C_1$-$C_8$-Alkylgruppen wie in Propin, 1-Butin, 2-Butin, 1-Hexin und 1-Octin.

Hydroxyalkylgruppen als Substituenten der Alkine sind bevorzugt Hydroxy-$C_1$-$C_4$-alkylgruppen, wie in 1-Butin-3-ol, 1,4-Butindiol und Propargylalkohol. Von den Arylgruppen-tragenden Alkinen sind Phenylalkine bevorzugt, z. B. Phenylacetylen und Diphenylacetylen. Die Arylgruppen können unter den Reaktionsbedingungen inerte Substituenten tragen wie Halogen, insbesondere Chlor, Alkoxy, insbesondere Methoxy, und Alkyl, insbesondere $C_1$-$C_4$-Alkyl. Weiter hin kommen Alkine in Betracht, die Trialkylsilylgruppen tragen, wie Trimethylsilylacetylen.

Bevorzugt sind solche Alkine, in denen mindestens einer der Substituenten $R^1$ und $R^2$ für Wasserstoff steht. Acetylen ist besonders bevorzugt.

Die Umsetzung des Alkins zum Butyrolacton erfolgt in Gegenwart von CO und Wasser:

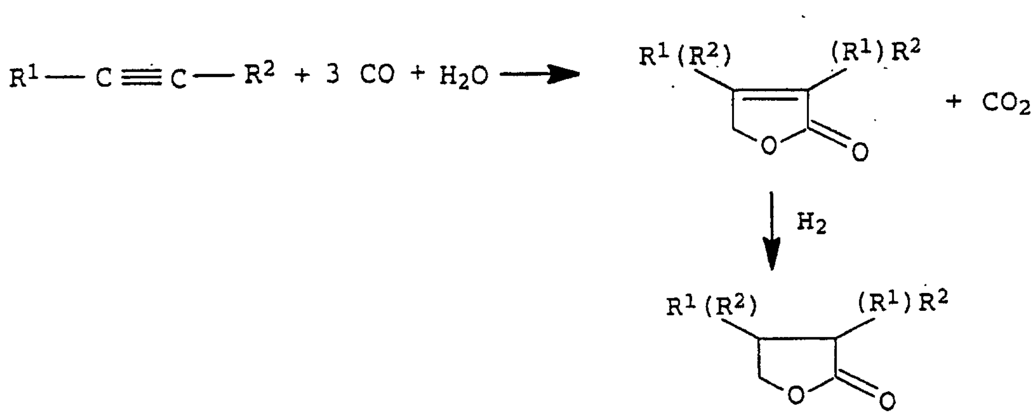

Bevorzugt werden 2 bis 50 Äquivalente Wasser je Äquivalent Alkin eingesetzt. Es ist empfehlenswert mindesten 4 Äquivalente CO je Äquivalent Alkin zu verwenden. CO kann auch im Überschuß eingesetzt werden, wobei jedoch in der Regel nicht mehr als ein 50facher Überschuß, bezogen auf das Alkin, an CO verwendet werden sollte, da noch größere Überschüsse keine erkennbaren technischen Vorteile bringen.

Die Carbonylierung kann unter den in WO 97/07111 beschriebenen Temperatur- und Druckbedingungen durchgeführt werden. Es hat sich jedoch als vorteilhaft erwiesen, bei der Durchführung des erfindungsgemäßen Verfahrens eher höhere Drücke anzuwenden. Demgemäß wird die Carbonylierung in der Regel bei Temperaturen von 50 bis 250°C, vorzugsweise 80 bis 150°C und insbesondere 100 bis 140°C und bei Drücken von 20 bis 300, vorzugsweise 100 bis 250 und insbesondere 170 bis 230 bar durchgeführt.

Die erfindungsgemäß zu verwendenden Katalysatoren sind in der Lage das Wassergasgleichgewicht gemäß der folgenden Gleichung einzustellen (water-gas-shift catalysts, s. Parshall et al., Homogeneous Catalysis, Wiley, 2. Auflage 1992, Kapitel 5.7):

$$CO + H_2O \rightarrow H_2 + CO_2$$

Der freiwerdende Wasserstoff kann so das zunächst gebildete 2(5H)-Furanon zum Butyrolacton hydrieren.

Durch Zusatzstoffe läßt sich die Aktivität der eingesetzten Katalysatoren steigern. Zu diesen Verbindungen gehören Amine. Hierzu kommen primäre, sekundäre und tertiäre Alkylamine und Cycloalkylamine ebenso in Betracht wie stickstoffhaltige Heterocyclen. Im einzelnen sind z.B. Methylamin, Ethylamin, Anilin, Diethylamin, Triethylamin, Tributylamin, Trioctylamin, Pyridin, Chinolin, Isochinolin und Dimethylaminopyridin zu nennen. Weiterhin zeigen sich

Ammoniumsalze wie Triethylammoniumhydrochlorid, Tetraethylammoniumchlorid, Tetrabutylammoniumacetat, Tetrabutylammoniumnitrat und Tetrabutylammoniumhydroxid wirksam.

Weiterhin können als Zusatzstoffe Halogenide eingesetzt werden. Im einzelnen kommen Alkalimetall- und Erdalkalimetallhalogenide wie NaCl, NaBr, NAI, KCl, KI, $CaCl_2$, $CaBr_2$, $CaI_2$ sowie Halogenide mit organischen Kationen wie Tetramethylammoniumchlorid, Tetramethylammoniumbromid, Tetramethylammoniumiodid, Tetrabutylammoniumchlorid, Tetrabutylammoniumbromid und Tetrabutylammoniumiodid in Betracht. Bevorzugte Halogenide sind die Iodide.

Auch Polymerisationsinhibitoren für olefinisch ungesättigte organische Verbindungen wie Hydrochinonmonomethylether, 2,6-Di-tert.-butyl-4-methylphenol und Phenothiazin kommen als Zusatzstoffe in Betracht.

Alle diese Zusatzstoffe haben keinen störenden Einfluß auf die Wiedergewinnung des Katalysators.

Die Menge an eingesetztem Zusatzstoff ist in weiten Grenzen variabel und kann zwischen 0,1 und 10 000 mol Zusatzstoff je Mol Katalysator liegen. Bevorzugt werden 0,5 bis 5 mol je Mol Katalysator eingesetzt. Es können ein oder mehrere Zusatzstoffe in einer Reaktion eingesetzt werden. Bevorzugt werden für das erfindungsgemäße Verfahren Amine, insbesondere tertiäre Amine zugesetzt.

Das erfindungsgemäße Verfahren wird entweder kontinuierlich oder diskontinuierlich bevorzugt in der Flüssigphase und bevorzugt in einem unter Reaktionsbedingungen inerten organischen Lösungsmittel durchgeführt, beispielsweise unter Zusatz von Ethern, wie Diethylether, Methyl-tert-butylether, Tetrahydrofuran und 1,4-Dioxan, Alkanole wie Methanol, Ethanol und Isopropanol, Kohlenwasserstoffen wie Pentan, Hexan und Cyclohexan, weiterhin Chlorkohlenwasserstoffen wie Methylenchlorid, Chloroform und Dichlorethan. Die Menge der Lösungsmittel beträgt in der Regel 5 bis 95 Gew.-% Lösungsmittel, bezogen auf den Reaktionsansatz. Das erfindungsgemäße Verfahren wird in druckfesten Reaktoren, wie Rühr-, Rohr- oder Schlaufenreaktoren kontinuierlich oder diskontinuierlich durchgeführt.

Im erfindungsgemäßen Verfahren kann somit der für die Synthese von γ-Butyrolactonen aus Alkinen und CO als Katalysator eingesetzte Rhodium-Komplex durch einfache Ausfällung aus dem Reaktionsaustrag unter hydrierenden Bedingungen mehrfach und jeweils nahezu quantitativ wiedergewonnen und mit praktisch gleicher Aktivität und Selektivität erneut eingesetzt werden.

Beispiele

Beispiel 1 (Vergleich)

In 400 ml Dioxan wurden 672 mg (2,6 mmol) $Rh(CO)_2acac$, 2,25 g (22,3 mmol) Triethylamin und 32,8 g (1,8 mol) Wasser vorgelegt. Bei Raumtemperatur preßte man unter Rühren 8,2 1 (0,31 mol) Acetylen auf, wobei sich ein Druck von 3,6 bar einstellte. Mit Kohlenmonoxid wurde der Druck dann auf 200 bar erhöht. Man erwärmte den Reaktionsansatz auf 120°C, wobei sich ein Druck von 248 bar einstellte, und rührte anschließend 2 h. Nach Abkühlen und Entspannen des Reaktors wurde der Austrag destillativ aufgearbeitet. Bezogen auf die eingesetzte Acetylenmenge waren 52 % 2(5H)-Furanon und 22 % Butyrolacton entstanden. Der Katalysator-haltige Destillationssumpf wurde in 400 ml Dioxan vorgelegt, mit 2,25 g (22,3 mmol) Triethylamin und 32,8 g (1,8 mol) Wasser versetzt. Bei Raumtemperatur preßte man unter Rühren 8,2 1 (0,31 mol) Acetylen auf, wobei sich ein Druck von 3,6 bar einstellte. Mit Kohlenmonoxid wurde der Druck dann auf 200 bar erhöht. Man erwärmte den Reaktionsansatz auf 120°C, wobei sich ein Druck von 248 bar einstellte, und rührte anschließend 2 h. Nach Abkühlen und Entspannen des Reaktors wurde der Reaktionsaustrag destillativ aufgearbeitet und der Katalysator-haltige Destillationssumpf erneut eingesetzt. Insgesamt wurde dieses Vorgehen dreimal wiederholt, ohne frischen Katalysator zuzufügen. die jeweiligen Ausbeuten an Wertprodukt (angegeben ist die Summe aus 2(5H)-Furanon und Butyrolacton) sind in Tabelle 1 zusammengestellt.

Tabelle 1:

| Katalysator-Rückführung nach destillativer Aufarbeitung | | | | |
|---|---|---|---|---|
| Katalysator-Rückführung | 0. | 1. | 2. | 3. |
| Ausbeute an Wertprodukt [%] | 74. | 56 | 43 | 21 |

Beispiel 2

In 400 ml Dioxan wurden 672 mg (2,6 mmol) $Rh(CO)_2acac$, 2,25 g (22,3 mmol) Triethylamin und 32,8 g (1,8 mol) Wasser vorgelegt. Bei Raumtemperatur preßte man unter Rühren 8,2 1 (0,31 mol) Acetylen auf, wobei sich ein Druck von 3,6 bar einstellte. Mit Kohlenmonoxid wurde der Druck dann auf 200 bar erhöht. Man erwärmte den Reaktionssatz auf 120°C, wobei sich ein Druck von 248 bar einstellte, und rührte anschließend 2 h. Nach Abkühlen- und Entspannen des Reaktors wurde eine Probe entnommen und gaschromatographisch nach der Methode des inneren

Standards untersucht. Bezogen auf die eingesetzte Acetylenmenge waren 59 % 2(5H)-Furanon und 24 % Butyrolacton entstanden. Bei Raumtemperatur wurden anschließend 50 bar Wasserstoff aufgepreßt und der Reaktionsansatz im Autoklaven auf eine Temperatur von 200°C erwärmt. Dabei stellte sich ein Druck von 89 bar ein. Mit Wasserstoff wurde dann der Druck auf 200 bar erhöht und 1 h gerührt. Nach Abkühlen und Entspannen des Autoklaven wurde der Reäktionsaustrag filtriert. Im Filtrat fand sich nur 1 % des eingesetzten Rhodiums wieder. Der abfiltrierte Feststoff konnte erneut als Katalysator eingesetzt werden. Nach destillativer Aufarbeitung konnte Butyrolacton mit einer Ausbeute von 81 % bezogen auf die eingesetzte Acetylenmenge isoliert werden.

Beispiel 3

Die Reaktion wurde analog zu Beispiel 2 ausgeführt, der Reaktionsaustrag nach der Hydrierung jedoch zentrifugiert. Das Zentrifugat wurde destillativ aufgearbeitet. Der abgetrennte Feststoff wurde in 400 ml Dioxan vorgelegt und mit 2,25 g (22,3 mmol) Triethylamin und 32,8 g (1,8 mol) Wasser versetzt. Bei Raumtemperatur preßte man unter Rühren 8,2 1 (0,31 mol) Acetylen auf, wobei sich ein Druck von 3,6 bar einstellte. Mit Kohlenmonoxid wurde der Druck dann auf 200 bar erhöht. Man erwärmte den Reaktionsansatz auf 120°C, wobei sich ein Druck von 248 bar einstellte, und rührte anschließend 2 h. Nach Abkühlen und Entspannen des Reaktors wurde eine Probe entnommen und gaschromatographisch nach der Methode des inneren Standards untersucht.

Bei Raumtemperatur wurden anschließend 50 bar Wasserstoff aufgepreßt und der Reaktionsansatz im Autoklaven auf eine Temperatur von 200°C erwärmt. Dabei stellte sich ein Druck von 89 bar ein. Mit Wasserstoff wurde dann der Druck 200 bar erhöht und 1 h gerührt. Nach Abkühlen und Entspannnen des Autoklaven wurde der Reaktionsaustrag erneut zentrifugiert, um den Katalysator abzutrennen und wieder einzusetzen. Das Zentrifugat wurde destillativ aufgearbeitet. Die Sequenz Carbonylierung-Hydrierung-Katalysatorabtrennung-Carbonylierung wurde insgesamt fünfmal wiederholt, ohne frischen Katalysator zuzufügen. Die nach jedem Zyklus erhaltenen destillativen Ausbeuten an Butyrolacton - jeweils auf die eingesetzte Acetylenmenge bezogen - sind in der nachfolgenden Tabelle 2 aufgeführt:

| Katalysator-Rückführung | 0. | 1. | 2. | 3. | 4. | 5. |
|---|---|---|---|---|---|---|
| Ausbeute an Butyrolacton [%] | 81 | 79 | 82 | 83 | 78 | 81 |

## Patentansprüche

**1.** Verfahren zur Herstellung von Butyrolactonen der allgemeinen Formel I

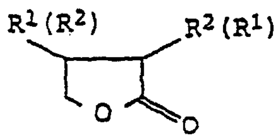

I,

in der die Substituenten $R^1$ und $R^2$ für Wasserstoff, Alkyl-, Hydroxyalkyl- oder gegebenenfalls mit Halogen, Alkoxy und/oder Alkyl substituierte Aryl- und Trialkylsilylgruppen stehen, durch Umsetzung von Alkinen der allgemeinen Formel II

$$R^1—C\equiv C—R^2 \qquad \text{II,}$$

in der $R^1$ und $R^2$ die oben angegebene Bedeutung haben, mit Kohlenmonoxid und Wasser in Gegenwart eines homogenen Rhodiumkatalysators unter Drücken von 20 bis 300 bar und Hydrierung der intermediär gebildeten, noch nicht hydrierten 2(5H)-Furanone, **dadurch gekennzeichnet, daß** man

a) das Reaktionsgemisch der Carbonylierung mit Wasserstoff bei Temperaturen von 150 bis 250°C und Drükken von 100 bis 300 bar umsetzt,

b) den ausgefallenen Katalysator abtrennt, und in die Carbonylierungsreaktion zurückführt und

c) aus dem vom Katalysator befreiten Reaktionsgemischen das Butyrolacton durch Destillation gewinnt.

**2.** Verfahren nach Anspruch 1, **dadurch gekennzeichnet, daß** man die Hydrierung (a) bei Temperaturen von 180 bis 230°C und Drücken von 150 bis 250 bar durchführt.

**3.** Verfahren nach Anspruch 1, **dadurch gekennzeichnet, daß** man den Katalysator in der Stufe (b) durch Filtrieren, Dekantieren oder Zentrifugieren gewinnt.

**4.** Verfahren nach Anspruch 1, **dadurch gekennzeichnet, daß** man die Schritte (a) bis (c) kontinuierlich durchführt.

**5.** Verfahren nach Anspruch 1, **dadurch gekennzeichnet, daß** man die Carbonylierungsreaktion mit mindesten 4 Äquivalenten CO und mindestens 2 Äquivalenten Wasser durchführt.

**6.** Verfahren nach Anspruch 1, **dadurch gekennzeichnet, daß** man die Carbonylierung in Gegenwart eines Amins oder eines Ammoniumsalzes durchführt.

**7.** Verfahren nach Anspruch 1, **dadurch gekennzeichnet, daß** man die Carbonylierung in Gegenwart eines Halogenids durchführt.

**8.** Verfahren nach Anspruch 1, **dadurch gekennzeichnet, daß** man die. Carbonylierung in Gegenwart eines Polymerisationsinhibitors durchführt.

**9.** Verfahren nach Anspruch 1, **dadurch gekennzeichnet, daß** man als Alkin Acetylen verwendet.

**10.** Verfahren nach Anspruch 1, **dadurch gekennzeichnet, daß** man die Umsetzung mit Kohlenmonoxid und Wasser bei Drücken von 170 bis 230 bar durchführt.

## Claims

**1.** A process for producing butyrolactones of the general formula I

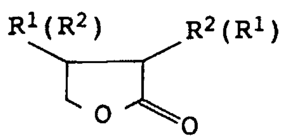

I,

where $R^1$ and $R^2$ are each hydrogen, alkyl, hydroxyalkyl, halogen-, alkoxy- and/or alkyl-substituted or unsubstituted aryl or halogen-, alkoxy- and/or alkyl-substituted or unsubstituted trialkylsilyl, by reacting alkynes of the general formula II

$$R^1—C\equiv C—R^2 \qquad II,$$

where $R^1$ and $R^2$ are each as defined above, with carbon monoxide and water in the presence of a homogeneous rhodium catalyst under pressures from 20 to 300 bar and hydrogenating the unhydrogenated 2(5H)-furanone intermediates, which comprises

a) reacting the carbonylation reaction mixture with hydrogen at from 150 to 250°C and from 100 to 300 bar,

b) removing the precipitated catalyst and returning it into the carbonylation reaction, and

c) subjecting the catalyst-free reaction mixture to a distillation to recover the butyrolactone.

**2.** A process as claimed in claim 1, wherein the hydrogenation (a) is carried out at from 180 to 230°C and from 150 to 250 bar.

**3.** A process as claimed in claim 1, wherein the recovering of the catalyst in step (b) is effected by filtration, decanting or centrifugation.

**4.** A process as claimed in claim 1, wherein steps (a) to (c) are carried out continuously.

**5.** A process as claimed in claim 1, wherein the carbonylation reaction is carried out with not less than 4 equivalents of CO and not less than 2 equivalents of water.

**6.** A process as claimed in claim 1, wherein the carbonylation is carried out in the presence of an amine or of an ammonium salt.

**7.** A process as claimed in claim 1, wherein the carbonylation is carried out in the presence of a halide.

**8.** A process as claimed in claim 1, wherein the carbonylation is carried out in the presence of a polymerization inhibitor.

**9.** A process as claimed in claim 1, wherein the alkyne used is acetylene.

**10.** A process as claimed in claim 1, wherein the reaction with carbon monoxide and water is carried out at from 170 to 230 bar.

**Revendications**

**1.** Procédé de préparation de butyrolactones de formule générale I

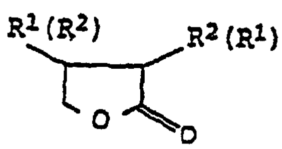

I,

dans laquelle les substituants $R^1$ et $R^2$ sont des atomes d'hydrogène ou des groupes alkyle, hydroxyalkyle, ou des groupes aryle et trialkylsilyle éventuellement substitués par des substituants halogéno, alcoxy et/ou alkyle, par réaction d'alcynes de formule générale II

$$R^1—C\equiv C—R^2 \qquad II$$

dans laquelle $R^1$ et $R^2$ ont les significations données ci-dessus, avec du monoxyde de carbone et de l'eau, en présence d'un catalyseur homogène au rhodium sous des pressions de 20 à 300 bar, et hydrogénation des 2(5H)-furannones formées d'une manière intermédiaire et non encore hydrogénées, **caractérisé en ce que**

a) on fait réagir le mélange réactionnel de la carbonylation avec de l'hydrogène à des températures de 150 à 250°C et sous des pressions de 100 à 300 bar,
b) on isole le catalyseur qui précipite, et on le renvoie dans la réaction de carbonylation, et
c) on obtient la butyrolactone par distillation à partir du mélange réactionnel débarrassé du catalyseur.

**2.** Procédé selon la revendication 1, **caractérisé en ce qu'**on procède à l'hydrogénation (a) à des températures de 180 à 230°C et sous des pressions de 150 à 250 bar.

**3.** Procédé selon la revendication 1, **caractérisé en ce qu'**on récupère le catalyseur dans l'étape (b) par filtration,

décantation ou centrifugation.

4. Procédé selon la revendication 1, **caractérisé en ce qu'**on met en oeuvre les étapes (a) à (c) d'une manière continue.

5. Procédé selon la revendication 1, **caractérisé en ce qu'**on met en oeuvre la réaction de carbonylation avec au moins 4 équivalents de CO et au moins 2 équivalents d'eau.

6. Procédé selon la revendication 1, **caractérisé en ce qu'**on met en oeuvre la carbonylation en présence d'une amine ou d'un sel d'ammonium.

7. Procédé selon la revendication 1, **caractérisé en ce qu'**on met en oeuvre la carbonylation en présence d'un halogénure.

8. Procédé selon la revendication 1, **caractérisé en ce qu'**on met en oeuvre la carbonylation en présence d'un inhibiteur de polymérisation.

9. Procédé selon la revendication 1, **caractérisé en ce qu'**on utilise en tant qu'alcyne l'acétylène.

10. Procédé selon la revendication 1, **caractérisé en ce qu'**on met en oeuvre la réaction avec le monoxyde de carbone et l'eau sous des pressions de 170 à 230 bar.